(19) Europäisches Patentamt / European Patent Office / Office européen des brevets

(11) **EP 4 571 286 A1**

(12) **EUROPÄISCHE PATENTANMELDUNG**

(43) Veröffentlichungstag:
18.06.2025  Patentblatt 2025/25

(21) Anmeldenummer: 23216329.5

(22) Anmeldetag: **13.12.2023**

(51) Internationale Patentklassifikation (IPC):
*G01N 3/08* (2006.01)

(52) Gemeinsame Patentklassifikation (CPC):
**G01N 3/068; G01N 3/08; G01N 33/36;**
G01N 2203/0003; G01N 2203/0017;
G01N 2203/0037; G01N 2203/0252;
G01N 2203/0282; G01N 2203/0647;
G01N 2203/0676; G01N 2203/0682

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **KOB GmbH**
**67752 Wolfstein (DE)**

(72) Erfinder:
• **Merkt, Thomas**
**67752 Wolfstein (DE)**
• **Haag, Torsten**
**67749 Offenbach Hundheim (DE)**
• **Buchholz, Florian**
**66869 Schellweiler (DE)**

(74) Vertreter: **Paul Hartmann AG**
**Patents & Licensing**
**Paul-Hartmann-Straße 12**
**89522 Heidenheim (DE)**

(54) **PRÜFGERÄT UND VERFAHREN ZUR BESTIMMUNG DER DEHNUNGSEIGENSCHAFTEN BAHNFÖRMIGER MATERIALIEN**

(57)   Die vorliegende Erfindung betrifft ein Prüfgerät zur Bestimmung der Dehnungseigenschaften von bahnförmigem, insbesondere textilem Material. Das Prüfgerät umfasst einen ersten Bereich, in dem das bahnförmige Material in einem vorzugsweise gewickelten Lagerzustand bereitgestellt wird, und einen dritten Bereich, welcher ein Zugwerk, eine Wickelwalze, und einen Längenmesssensor umfasst. Optional umfasst das Prüfgerät einen zweiten Bereich, in dem ein Bestimmen der ungedehnten Länge des bahnförmigen Materials erfolgen kann. Das Zugwerk ist dazu ausgestaltet, das bahnförmige Material zur Wickelwalze weiterzuleiten. Das bahnförmige Material wird unter Einwirkung einer vorherbestimmten Zugkraft zwischen Zugwerk und Wickelwalze gedehnt und danach unter Wirkung der Zugkraft auf die Wickelwalze aufgewickelt. Die gedehnte Länge wird mittels des Längenmesssensors bestimmt.

Abbildung 2

EP 4 571 286 A1

**Beschreibung**

Die vorliegende Erfindung betrifft ein Prüfgerät und ein Verfahren zur Bestimmung der Dehnungseigenschaften bahnförmiger Materialien.

Die Überprüfung der produktspezifischen Parameter Dehnung und Rückzug stellt einen wichtigen Bestandteil der internen Prozess- und Qualitätskontrolle für bahnförmige Materialien dar. Die Dehnung eines bahnförmigen Materials beschreibt hierbei, in welchem Verhältnis es sich, in Bezug auf ihre Ausgangslänge, unter Belastung einer bestimmten Kraft längt. Die Dehnung ist der Quotient aus Längenänderung und Ausgangslänge. Zur Bestimmung des Rückzuges wird die Binde mit der konstanten Kraft, die von der Breite des bahnförmigen Materials abhängt, für eine vom Nutzer vorbestimmte Zeitdauer belastet. Anschließend wird ermittelt, inwieweit sich bahnförmiges Material bei einem Wegfall der Zugkraft wieder in Längsrichtung zusammenzieht. Somit ist der Rückzug ein Maß für das elastisch-plastische Verformungsverhalten des bahnförmigen Materials.

Gegenwärtig beruhen Prüfanweisungen für bahnförmige Materialien, insbesondere für Binden, auf der Normen DIN 61632 und DIN EN 1773.

DIN EN 1773 beschreibt das Verfahren zur Bestimmung der Breite und Länge von textilen Flächengebilden im entspannten Zustand. Anforderungen an eine textilelastische Idealbinde und dazugehörige Prüfmethoden, welche auch die Bestimmung der Dehnung und des Rückzuges einschließen, werden in der Norm DIN 61632 dargestellt. Als Idealbinden werden in der Norm Binden bezeichnet, "die durch ihre poröse Webart und die Verwendung von Kreppzwirnen in der Kette der Länge nach elastisch entweder mit Webkanten (webkantig) oder mit nicht ausfransenden Dreherleisten (schlingkantig) hergestellt sind".

Die Bestimmung der ungedehnten Länge erfolgt mit Hilfe eines kalibrierten Lineals und einem Messtisch mit einer ebenen und glatten Oberfläche, der größere Dimensionen in Länge und Breite als das zu prüfende bahnförmige Material besitzt. Das bahnförmige Material wird spannungslos auf einem Tisch ausgebreitet und die Endkanten werden gekennzeichnet. Anschließend wird unter der Verwendung von am Tisch angebrachten Messmarken, welche jeweils in einem Abstand von 1 m zueinander aufgebracht sind, das bahnförmige Material in Abständen von 1 m markiert. Die Restlänge wird mit dem kalibrierten Lineal bestimmt. Als Gesamtlänge der Probe ergibt sich die Summe aus den 1 m-Teilstücken und der Restlänge des bahnförmigen Materials.

Die Ermittlung der gedehnten Länge und der Restlänge erfolgt mittels Kraftdehnungsanlage nach DIN 61632. Diese Kraftdehnungsanlage besteht aus einer festen und beweglichen Einklemmung, in denen jeweils der Anfang und das Ende des bahnförmigen Materials fixiert werden. Eine Zugkraft wird zwischen den Klemmen erzeugt.

Danach wird die Länge des gedehnten und ungedehnten bahnförmigen Materials bestimmt.

Die Verwendung dieser Kraftdehnungsanlagen erfordern einen hohen manuellen Arbeitsaufwand und die dauerhafte Bindung des Bedienpersonals während des Prüfprozesses. Infolge von konstruktionsbedingten Störeinflüssen durch Reibung und damit einhergehenden systematischen Messfehlern kann nicht sichergestellt werden, dass das bahnförmige Material mit der vorgeschriebenen Zugkraft gedehnt werden. Somit ist die produktionsbegleitende Prozesskontrolle und die abschließende Qualitätskontrolle des bahnförmigen Materials mit Unsicherheiten behaftet.

Aufgabe ist es daher ein Prüfgerät zur Verfügung zu stellen, das die Dehnungseigenschaften von bahnförmigem Material effizienter bestimmen kann.

Diese Aufgabe wird gelöst durch ein Prüfgerät zur Bestimmung der Dehnungseigenschaften von bahnförmigem Material nach Anspruch 1 und ein Messverfahren nach Anspruch 9.

Das Prüfgerät umfasst einen ersten Bereich, in dem das bahnförmige Material in einem vorzugsweise gewickelten Lagerzustand bereitgestellt wird, optional einen zweiten Bereich, in dem eine Bestimmen der ungedehnten Länge des bahnförmigen Materials erfolgen kann und einen dritten Bereich. Der dritte Bereich umfasst ein Zugwerk, eine Wickelwalze, und einen Längenmesssensor. Das Zugwerk ist dazu ausgestaltet, das bahnförmige Material zur Wickelwalze weiterzuleiten. Das bahnförmige Material kann unter Einwirkung einer vorherbestimmten Zugkraft zwischen Zugwerk und Wickelwalze gedehnt werden. Die Wickelwalze ist dazu ausgestaltet, dass das bahnförmige Material unter Wirkung der Zugkraft aufgewickelt werden kann. Die gedehnte Länge des bahnförmigen Materials kann mittels des Längenmesssensors bestimmt werden.

Das Verfahren zur Bestimmung der Dehnungseigenschaften des bahnförmigen Materials umfasst die folgenden Schritte:

    a. Das zu prüfende bahnförmige Material wird in das Prüfgerät eingelegt.
    b. Das bahnförmige Material wird zumindest teilweise aus seinem gewickelten Lagerzustand abgewickelt und ein Anfangsstück des bahnförmigen Materials wird in die Wickelwalze eingelegt.
    c. Das bahnförmige Material wird dem Zugwerk vom ersten Bereich ausgehend zugeführt und dann mittels des Zugwerks zu der Wickelwalze weitergeführt.
    d. Im dritten Bereich zwischen dem Zugwerk und der Wickelwalze wird eine Zugkraft aufgebaut, so dass das bahnförmige Material dort unter Zugkraft geführt wird.
    e. Die Wickelwalze nimmt das bahnförmige Material unter Wirkung der Zugkraft auf.
    f. Mittels des Längenmesssensors wird die gedehnte Länge des bahnförmigen Materials bestimmt.

Da das erfindungsgemäße Verfahren unter Verwendung des erfindungsgemäßen Prüfgeräts automatisch ablaufen kann, hat es die Vorteile einer kürzeren Prüfungsdauer, einer geringerer Personalbindung und eines verringerten Auftretens von Fehlern. Des Weiteren hat das erfindungsgemäße Prüfgerät einen geringeren Platzbedarf als der herkömmliche Messaufbau.

Das bahnförmige Material umfasst Material, das sich aufgrund seiner Steifigkeit aufwickeln lässt. Das bahnförmige Material umfasst Bahnen aus Papier, Folien, Textilien oder aus anderem dünnem Material. Insbesondere umfasst das bahnförmige Material Binden aus textilem Material. Die Binden können aus Gewebe, Gewirke oder Vliesstoffen bestehen. Das bahnförmige Material kann kohäsiv sein, insbesondere können es kohäsive Binden sein. Das bahnförmige Material kann adhäsiv sein, insbesondere können es adhäsive Binden sein. Ebenfalls kann das bahnförmige Material Binden mit Schnittkanten und/oder Webkanten umfassen. Ebenso kann das bahnförmige Material Zinkleimbinden umfassen.

Das Zugwerk dient der Weiterleitung des bahnförmigen Materials. Gleichzeitig wird durch das Zugwerk die Zugkraft auf das bahnförmige Material, welches von Zugwerk zu Wickelwalze geleitet wird, eingeleitet. Die Zugkraft, die aufgebracht werden soll, kann bevorzugt variabel festgelegt werden. Das Zugwerk umfasst mindestens zwei Walzen. Bevorzugt ist mindestens eine Walze angetrieben. Die Walze kann mittels eines Servomotors mit Zahnriemenantrieb angetrieben werden. Bevorzugt ist mindestens eine der beiden Walzen so beschichtet, dass die Reibung erhöht wird. Besonders bevorzugt ist mindestens eine der beiden Walzen gummiert.

In einer bevorzugten Ausführungsform umfasst das Zugwerk eine erste Walze, welche bevorzugt gummiert und angetrieben ist, und zusätzlich eine obere Anpresswalze, welche mit der ersten Walze je einen Berührpunkt aufweist.

In einer besonders bevorzugten Ausführungsform ist das Zugwerk eine Dreiwalzensystem und umfasst eine erste Walze und eine zweite Walze, welche bevorzugt gummiert und angetrieben sind, und zusätzlich eine über der ersten und der zweiten Walze angeordnete Anpresswalze, welche mit der ersten und der zweiten Walze je einen Berührpunkt aufweist. Die erste Walze fördert das bahnförmige Material zur zweiten Walze. Die obere Anpresswalze ist vertikal verschiebbar zu den unteren Antriebswalzen ausgestaltet. Bevorzugt kann die obere Anpresswalze vertikal verschiebbar mittels eines oder mehrerer Pneumatikzylinder verschoben werden. Ebenso kann die Anpresswalze hydraulisch oder nur mittels Gewichtskraft verschoben werden.

Infolge der zusätzlichen zweiten Walze ist die Steifigkeit und die Abstützwirkung bei Dreiwalzensystemen höher als bei Ausführungsformen mit nur zwei Walzen. Damit können größere Andruckkräfte verwendet werden und das Zugwerk kann größere Zugkräfte zwischen Zugwerk und Wickelwalze einleiten, während gleichzeitig Schlupf zwischen dem zu prüfenden bahnförmigen Material und den Walzen vermieden wird.

Die Wickelwalze ist dazu ausgestaltet, dass das bahnförmige Material unter Wirkung einer definierten Zugkraft aufgewickelt wird.

Unter dem Wickeln versteht man einen Vorgang, bei dem ein Gegenstand, dessen Länge im Vergleich zu seinen beiden anderen Dimensionen sehr groß ist, in Form einer archimedischen Spirale aufgerollt wird. Hierbei entstehen im Wesentlichen zylindrische Geometrien.

Die zur Bestimmung der gedehnten Länge aufzubringende Zugkraft wird zwischen dem Zugwerk und der Wickelwalze aufgebaut. In Folge einer initialen Geschwindigkeitsdifferenz zwischen dem Zugwerk und der Wickelwalze wird das bahnförmige Material durch die daraus resultierende Kraftwirkung gedehnt. Ist die definierte Zugkraft erreicht, so wird sie konstant gehalten und das bahnförmige Material wird unter Wirkung dieser definierten Zugkraft gedehnt aufgewickelt.

Die Wickelwalze umfasst bevorzugt einen Motor.

Bevorzugt ist die Wickelwalze ein Zentrumswickler. Bei Zentrumswicklern wird nur die axial verlaufenden Wickelwelle angetrieben. Hierbei erfolgt die Übertragung des zum Wickeln benötigten Torsionsmomentes über den Kern, bzw. über die Hülse. Das aufzubringende Drehmoment wird vom Kern über die Wickellagen bis hin zur äußeren Lage übertragen, wobei die Übertragung der Bahnkraft über die Zugkraft der Lage und Schiebekräfte zwischen den Lagen erfolgt.

In einer bevorzugten Ausführungsform ist die Wickelwalze eine aufklappbare Walze mit Verzahnung zwischen den beiden Walzenhälften, in die an den Mittelflächen keilförmige Längsnuten eingefräst sind. Dadurch kann das bahnförmige Material beim Zuklappen der Walze eingeklemmt werden und ein Herausrutschen des bahnförmigen Materials aus der Klemmung insbesondere zu Beginn des Wickelprozesses vermieden werden. Die erste Walzenhälfte kann durch Schraubenverbindungen fest mit der Welle der Walze verbunden werden. Die zweite Walzenhälfte ist bevorzugt mit einem Scharnier an einer Flanschplatte einer Welle befestigt. Dadurch kann die erste Walzenhälfte zum Einlegen des bahnförmigen Materials hochgeklappt werden. Eine in das Scharnier integrierte Schenkelfeder kann die aufklappbare zweite Walzenhälfte für ein sicheres Einlegen des Anfangs des bahnförmigen Materials oben halten. Zum dauerhaften Verschließen der Walzenhälften während des Wickelprozesses können diese mit einem Schnellspannverschluss, welcher an der Frontseite der Walze montiert ist, gegeneinandergepresst werden. Somit wird eine konstante Normalkraft der Walzenhälften auf das bahnförmige Material ermöglicht.

In einer Ausführungsform besteht der mittig geteilte Walzenkörper der Wickelwalze aus Aluminium. Um den abrasiven Verschleiß der Walzenkörper zu minimieren, können die Oberflächen des Walzenkörpers eloxiert sein.

Anstelle der Wickelwalze kann auch ein Förderband zum Dehnen und Aufwickeln des bahnförmigen Materials zur Verfügung gestellt werden. Ein Ende des bahnförmigen Materials wird mittels eines Klemmmechanismus an dem Förderband befestigt. Da zwischen der Umfangsgeschwindigkeit der Walzen des Zugwerkes und der Bahngeschwindigkeit des Förderbandes eine Differenz vorliegt, resultiert eine in Längsrichtung des bahnförmigen Materials wirkende Zugkraft, welche das Gewebe dehnt. Da die Zugkraft abhängig von der Breite des bahnförmigen Materials ist und zudem während des Aufwickelns bevorzugt konstant gehalten wird, muss die Zugkraft kontinuierlich bestimmt werden. Die Messung der Zugkraft wird bevorzugt mittels eines in das Förderband integrierten Kraftmessers umgesetzt. Das bahnförmige Material wird unter Last der Zugkraft auf das Förderband aufgewickelt und so über die ganze Länge gleichmäßig mit einer konstanten Zugkraft belastet und gedehnt. Die Bestimmung der gedehnten Länge des bahnförmigen Materials kann mit Hilfe des Antriebs des Förderbandes erfolgen, mit dem sich die gedehnte Länge des bahnförmigen Materials durch die Anzahl der Umdrehungen bzw. der überstrichenen Inkremente ermitteln lässt.

Ebenfalls kann die Wickelwalze durch einen zweiachsigen Portalroboter in Kombination mit Umlenkrollen ersetzt werden. Der Portalroboter umfasst einen Greifer. Mittels des Greifers kann das bahnförmige Material gegriffen werden und in einer Fläche, welche über die Achsen des Portalroboters bestimmt wird, beliebig positioniert werden. Eine erste Menge der Umlenkrollen sind bevorzugt so angeordnet, dass ihre Achsen parallel übereinander ausgerichtet sind. Eine zweite Menge der Umlenkrollen sind bevorzugt so angeordnet, dass ihre Achsen parallel übereinander ausgerichtet sind. Die erste und zweite Menge der Umlenkrollen werden so nebeneinander angeordnet, dass ihre Achsen parallel zueinander ausgerichtet sind. Die Umlenkrollen sind auf einem Rollengerüst angeordnet. Das bahnförmige Material wird mittels des Portalroboters mäanderförmig um die Umlenkrollen geführt. Nachdem das bahnförmige Material über die gesamte Länge gedehnt wurde, kann die gedehnte Länge anhand des Verfahrweges des Portalroboters bestimmt werden.

Da die Zugkraft auf die Binde während dieses Vorganges konstant sein muss, umfasst das Prüfgerät bevorzugt einen Kraftmesser. Der Kraftmesser misst die Zugkraft, die auf das bahnförmige Material wirkt.

Der Kraftmesser ist bevorzugt über den an dem horizontal reibungsarm gelagerten Lagerbock der Wickelwalze mit einem Maschinengestell verbunden. Das Maschinengestell ist das Grundgestell, auf dem ein Prüfgerät aufgebaut ist. Um die Zugkraft ohne Störeinflüsse zu bestimmen, wird der Kraftmesser zwischen dem Maschinengestell und Lagerung der Wickelwalze eingebaut. Aus diesem Grund muss die horizontale Lagerung der Wickelwalze möglichst reibungsfrei ausgeführt sein, daher wird bevorzugt ein Luftlagerschlitten eingesetzt. Werden Abweichungen von der eingestellten Zugkraft mittels des Kraftmessers gemessen, so kann die Zugkraft durch Änderungen der Walzengeschwindigkeiten geregelt werden.

Die gedehnte Länge der Binde kann mittels der Durchmesser des Wickels und der Anzahl der Wickellagen bestimmt werden. In einer bevorzugten Ausführungsform ist der Längenmesssensor ein optischer Sensor. Der optische Sensor kann den Durchmesser des Wickels auf der Wickelwalze nach der Dehnung des bahnförmigen Materials und die gedehnte Materialstärke vermessen. Die berührungslose Längenbestimmung mittels des optischen Sensors kann auf dem Laser-Doppler Messprinzips beruhen. Bei diesem Messprinzip wird ein Streifenmuster mittels zweier Laserstrahlen und einer Linse auf dem zu vermessenden Objekt erzeugt. In Folge der Bewegung der zu vermessenden Objektoberfläche wird das in einen Detektor zurückgestreute Licht in seiner Intensität moduliert. Die Frequenz der Intensitätsmodulation entspricht gerade der Laser-Dopplerfrequenz und ist proportional zur Geschwindigkeit des betrachteten Objektes. Mit Hilfe eines Fotodetektors wird das gestreute Licht in ein äquivalentes elektrisches Signal umgewandelt, mit dem die aktuelle Geschwindigkeit und insbesondere die Weglänge des Objektes berechnet werden kann. Aus der Weglänge kann unmittelbar die Länge des gedehnten, bahnförmigen Materials auf der Wickelwalze bestimmt werden.

In einer alternativen Ausführungsform kann die Länge des gedehnten bahnförmigen Materials mittels eines ersten Messradsystems an der Wickelwalze ermittelt werden. Das erste Messradsystem verfügt bevorzugt über ein Messrad und einen Drehgeber. Das Messrad berührt das bewegte bahnförmige Material bei der Längenmessung direkt und dreht sich entsprechend der Bahngeschwindigkeit. Durch die Anzahl der Umdrehungen des Messrades, beziehungsweise der somit überstrichenen Inkremente, kann die zurückgelegte Wegstrecke des bahnförmigen Materials bestimmt werden. In einer weiteren Ausführungsform wird das erste Messradsystem an der Wickelwalze und der optische Sensor kombiniert, um die Präzision der Messung zu erhöhen.

In einer Ausführungsform der Erfindung kann das Prüfgerät ein Messradsystem mit einem Drehgeber und einem Messrad zu Bestimmung der ungedehnten Länge des bahnförmigen Materials im zweiten Bereich umfassen. Das Messradsystem ist dabei bevorzugt am Zugwerk an dem bahnförmigen Material angeordnet und wird durch Reibschluss angetrieben.

Das Messradsystem kann darüber hinaus einen Federarm umfassen. Das Messradsystem ist über diesen Federarm an einer Halterung befestigt. Die ungedehnte Länge des bahnförmigen Materials wird mittels des Messradsystems bevorzugt an der ersten Walze des Zugwerkes bemessen. Durch den Federarm kann das Messradsystem um die Achse der Halterung geschwenkt werden. Dies erleichtert das Einlegen des bahnförmigen Materials in das Prüfgerät. Mittels des Federarms ist es möglich die Anpresskraft des Messrades auf das bahnförmige Material einzustellen.

Das Messrad weist bevorzugt eine Oberfläche auf, durch die Schlupf vermieden wird. Beispielsweise weist das Messrad eine geriffelte Oberfläche auf. Alternativ kann das Messrad eine gummierte Oberfläche haben.

Des Weiteren kann das Prüfgerät ein Abzugswerk im ersten Bereich umfassen.

Das Abzugswerk bringt die Kraft auf, um das bahnförmige Material aus seinem gewickelten Lagerzustand in eine zumindest teilweise abgewickelte Materialbahn zu überführen. Beispielsweise wird das bahnförmige Material von einer Lagerrolle, auf die das bahnförmige Material aufgewickelt ist, mittels des Abzugswerks abgezogen. Diese Kraft beträgt beispielsweise bei kohäsiven Binden, deren Lagen im aufgewickelten Zustand aneinanderhaften, bis zu 165 cN/cm.

Das Abzugswerk umfasst mindestens zwei Walzen. Die Achsen der Walzen des Abzugswerks sind parallel zueinander ausgerichtet. Die Walzen können beispielsweise massive Stahlwalzen oder leichtläufige Walzen mit integrierten Rillenkugellagern und einem Walzenkörper aus einem Aluminium-Hohlprofil für minimale Massenträgheitsmomente sein. Bevorzugt bestehen die Walzen aus Aluminium.

Für das Abzugswerk werden bevorzugt zwei verschiedene Walzentypen eingesetzt.

In einer bevorzugten Ausführungsform umfasst das Abzugswerk ein Zweiwalzensystem, welches eine erste und eine zweite Walze umfasst, deren Mittelachsen parallel zueinander verlaufen. Dadurch entsteht ein Walzspalt in der Berührungslinie zwischen den Walzen.

Bevorzugt ist die erste Walze des Abzugswerks eine angetriebene Walze, mit der das Motordrehmoment auf das bahnförmige Material übertragen wird. Da das bahnförmige Material die angetriebene Walze nicht umschlingt, ist die zweite Walze eine Anpresswalze. Die Anpresswalze ist bevorzugt oberhalb der ersten Walze angeordnet ist und drückt das bahnförmige Material im Walzspalt an die angetriebene Walze. Die Anpresswalze ist vertikal verfahrbar und frei drehbar gelagert, so dass die Anpresswalze auf die erste Walze gedrückt werden kann. Bevorzugt wird die Anpresswalze mittels eines Pneumatikzylinders verschoben. Alternativ kann die Anpresswalze mittels Linearachse oder einer Spindelachse verschoben werden. Die Normalkraft der Anpresswalze auf die angetriebene Walze ist proportional zur Reibkraft zwischen dem bahnförmigen Material und der Antriebswalze, welche für das Abziehen des bahnförmigen Materials, beispielsweise bei den kohäsiven Binden, benötigt wird. Diese Normalkraft zwischen der ersten Walze und zweiten Walze, welche mittels des Pneumatikzylinders aufgebracht wird, muss ausreichend groß sein, sodass kein Schlupf zwischen angetriebener Walze und dem bahnförmigen Material auftritt. Andernfalls kann es dazu kommen, dass trotz drehender Antriebswalze das bahnförmige Material nicht abgewickelt wird und das bahnförmige Material zwischen Abzugswerk und Zugwerk unter Zug geführt wird. Dadurch wirkt im dritten Bereich eine größere Zugkraft auf das bahnförmige Material und die Messung wird verfälscht.

Bevorzugt ist die erste Walze des Abzugswerks gummiert, um die Reibung zwischen dem abgezogenen bahnförmigen Material und der ersten Walze zu erhöhen und so Schlupf zu vermeiden.

Bevorzugt besteht die Oberfläche der zweiten Walze des Abzugswerks aus eloxiertem Aluminium.

Bevorzugt ist das Abzugswerk Teil einer Abwickelvorrichtung. Bevorzugt umfasst die Abwickelvorrichtung des Weiteren zumindest einen Dorn oder eine Ablagemulde zur Aufnahme des bahnförmigen Materials. Bahnförmiges Material, das auf Hülsen aufgewickelt ist, kann auf den Dorn aufgesteckt werden. Die Verwendung von Hülsen ist vor allem bei kohäsivem bahnförmigem Material, wie kohäsiven Binden, üblich. Mittels des Abzugswerk kann das bahnförmige Material von dem Dorn gezogen werden.

Wird das bahnförmige Material ohne innenliegende Hülse gewickelt, so wird bevorzugt eine Ablagemulde verwendet. Mittels eines Stegs wird verhindert, dass das bahnförmige Material beim Abwickelprozess aus der Mulde gezogen wird.

Nachdem das bahnförmige Material mittels des Abzugswerks abgewickelt wird, wird das bahnförmige Material bevorzugt über einen geregelten Durchhang dem Zugwerk zugeführt. Mit Hilfe des geregelten Durchhanges kann das bahnförmige Material berührungslos und spannungsarm dem Zugwerk zugeführt werden. Damit erhöht sich die Messgenauigkeit, da sichergestellt ist, dass im dritten Bereich nur die vorgesehene Zugkraft auf das bahnförmige Material wirkt. Ebenso kann im zweiten Abschnitt die Länge des ungedehnten bahnförmigen Materials mittels des Messrads mit Drehgeber gemessen werden. Die Höhe des Durchhanges wird mit einem Durchhangsensor ermittelt und kann mit Hilfe einer Anpassung der Winkelgeschwindigkeit der Abzugswalzen konstant eingeregelt werden. Der Durchhangsensor ist bevorzugt ein optischer Sensor.

Im zweiten Abschnitt und im dritten Abschnitt kann neben der gedehnten Länge des bahnförmigen Materials auch die rückgewonnene Länge des bahnförmigen Materials bestimmt werden. Die rückgewonnene Länge ist die Länge, die das entspannte, bahnförmige Material ohne Krafteinwirkung nach einer Ruhephase aufweist, nachdem das bahnförmige Material mit einer definierten Zugkraft belastet wurde.

Um die rückgewonnene Länge nach der Dehnung eines bahnförmigen Materials zu ermitteln, kann das bahnförmige Material gedehnt auf der Wickelwalze ausharren. Bevorzugt harrt das bahnförmige Material für eine Zeit von einer Minute auf der Wickelwalze aus. Danach folgt eine Ruhephase. Das bahnförmige Material wird von der Wickelwalze abgewickelt und in entspanntem Zustand, bevorzugt in einer Ruhemulde, gelagert.

Das bahnförmige Material verharrt dort bevorzugt zwei Minuten. Nach der Ruhephase wird das bahnförmige Material vom Zugwerk in Richtung eines Abwickelgestells gefördert und mittels des Messradsystems in dem zweiten Abschnitt die rückgewonnene Länge des bahnförmigen Materials bestimmt. Bevorzugt wird die Zugkraft, welche zur Dehnung des bahnförmigen Materials einen konstanten und definierten Wert hat, mittels des Kraftmessers zeitaktuell ermittelt und durch eine Regelung der Antriebe der Walzen des Zugwerks und der Wickelwalze möglichst konstant gehalten.

Bevorzugt wird das Verfahren zur Ermittlung der gedehnten Länge des bahnförmigen Materials im Anschluss an das Verfahren zur Bestimmung der Dehnungseigenschaften des bahnförmigen Materials durchgeführt. Bevorzugt wird das bahnförmige Material zwischen den Messschritten nicht aus dem Prüfgerät entfernt.

Abbildung 1 zeigt eine erste Ausführungsform des erfindungsgemäßen Prüfgeräts

Abbildung 2 zeigt eine zweite Ausführungsform des erfindungsgemäßen Prüfgeräts

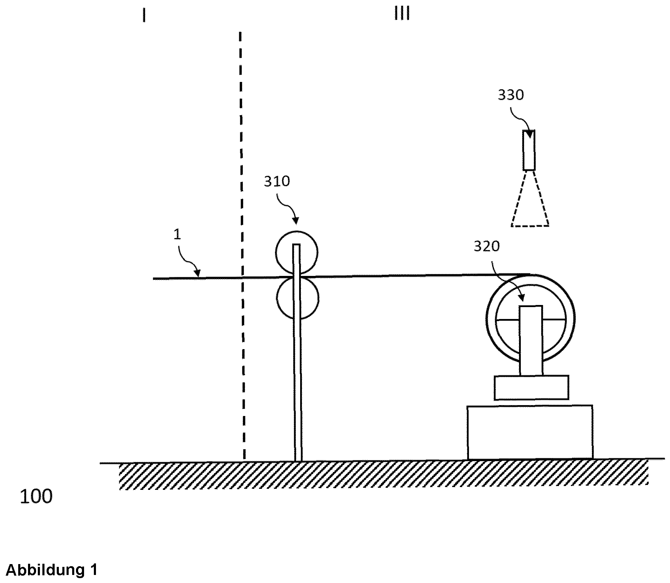

Die Ausführungsform des Prüfgeräts 100 der Abbildung 1 besteht unter anderem aus einem ersten Bereich I, in dem das bahnförmige Material 1 bereitgestellt wird. Der dritte Bereich III umfasst ein Zugwerk 310 und eine Wickelwalze 320.

Des Weiteren umfasst das Prüfgerät 100 einen ein Längenmesssensor 330 zur Bestimmung der gedehnten Länge des bahnförmigen Materials 1.

Das zu prüfende bahnförmige Material 1 wird in das Prüfgerät 100 eingelegt und teilweise abgerollt, sodass ein Anfangsstück des bahnförmigen Materials in die Wickelwalze 320 eingelegt werden kann. Das bahnförmige Material 1 wird vom ersten Bereich an das Zugwerk 310 weitergeleitet. Das Zugwerk 310 ist dazu ausgestaltet, das bahnförmige Material zur Wickelwalze 320 weiterzuleiten. Zwischen Zugwerk 310 und Wickelwalze 320 wird eine vorbestimmte Zugkraft angebracht, wodurch das bahnförmige Material 1 gedehnt wird. Gleichzeitig wird das gedehnte bahnförmige Material 1 und die Wickelwalze 320 weitergeführt. Die Wickelwalze 320 wickelt das bahnförmige Material 1 unter Wirkung der Zugkraft auf. Die gedehnte Länge des bahnförmigen Materials wird mittels des Längenmesssensors 330 bestimmt.

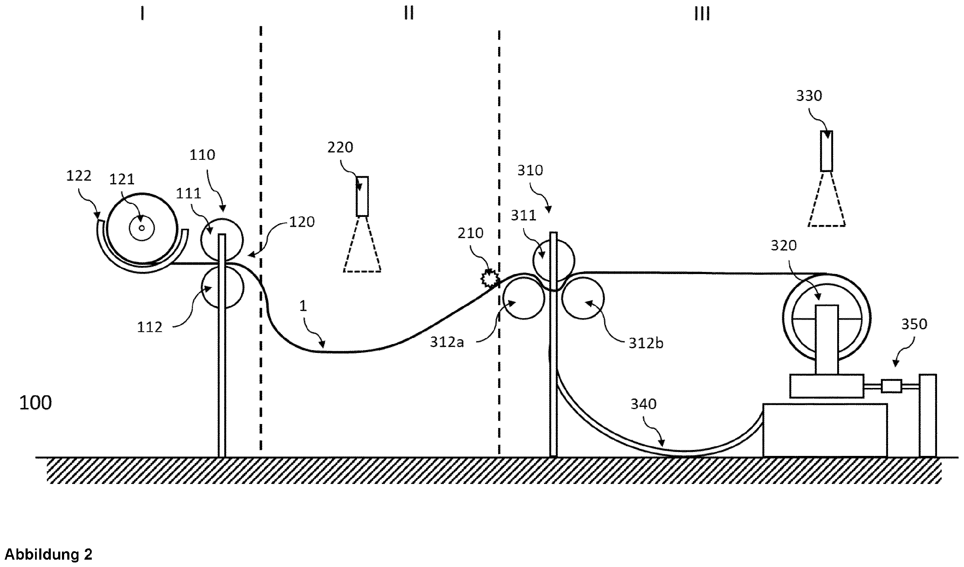

Die Ausführungsform des Prüfgeräts der Abbildung 2 besteht unter anderem aus einem ersten Bereich I, welcher eine Abwickelvorrichtung mit einem Abzugswerk 110 und einem Dorn 121 sowie einer Ablagemulde 122 umfasst, und einem dritten Bereich III umfassend ein Zugwerk 310 und eine Wickelwalze 320. Dabei ist das Zugwerk 310 zwischen Wickelwalze 320 und Abzugswerk 110 angeordnet. Im zweiten Bereich II ist ein Messradsystem 210 mit Messrad und Drehgeber an dem Zugwerk 310 angeordnet. Das Messradsystem 210 kann dazu verwendet werden die ungedehnte und die rückgewonnene Länge des bahnförmigen Materials 1 zu bestimmen.

Des Weiteren umfasst das Prüfgerät 100 einen ersten optischen Sensor 220 zur Bestimmung des Durchhangs des bahnförmigen Materials 1, welcher zwischen Abzugswerk 110 und Zugwerk 310 angeordnet ist und einen zweiten optischen Sensor 330, welcher dazu ausgestaltet ist, die gedehnte Länge des bahnförmigen Materials 1 auf der Wickelwalze 320 mittels des Umfangs auf der Wickelwalze 320 aufgewickelten bahnförmigen Materials 1 zu bestimmen.

Des Weiteren umfasst das Prüfgerät 100 eine Ruhemulde 340, die zwischen Zugwerk 310 und Wickelwalze 320 angeordnet ist, und ein Abwickelgestell zur Aufnahme des bahnförmigen Materials 1 nach der Prüfung der Dehnungseigenschaften (hier nicht dargestellt).

Das Abzugswerk 110 ist ein Zweiwalzensystem. Es besteht aus einer unteren angetriebenen Walze 112 und einer darüber angeordneten Anpresswalze 111. Die Achsen beider Walzen sind zueinander parallel ausgerichtet. Die untere Walze 112 wird über einen Servomotor angetrieben. Die Anpresswalze 112 ist über eine Führungsschiene vertikal verfahrbar und frei drehbar gelagert.

Die angetriebene Walze 112 des Abzugswerks ist gummiert.

Das Zugwerk 310 ist ein Dreiwalzensystem aus einer ersten angetriebenen Walze 312a und einer zweiten angetriebenen Walze 312b, sowie einer Anpresswalze 311. Dabei ist die Anpresswalze 311 mittig über den beiden angetriebenen Walzen 312a, 312b angeordnet. Die Anpresswalze 311 kann vertikal mittels Pneumatikzylinder verschoben werden. Die beiden angetriebenen Walzen 312a, 312b sind gummiert.

Die Wickelwalze 320 ist eine Zentrumswickler, welcher mittels eines Motors angetrieben wird. Die Wickelwalze 320 ist aufklappbar, so dass das bahnförmige Material 1 zwischen den Zylinderhälften der Walze eingeklemmt werden kann.

Die zu prüfende Binde wird in das Prüfgerät 100 eingelegt. Im ersten Schritt wird die zu prüfende Binde manuell in das Prüfgerät 100 eingelegt. Auf Hülsen gewickelten Binden 1 werden mit der Hülse auf den Dorn 121 aufgeschoben. Sollen hülsenlose Binden geprüft werden, wird der Dorn 121 weggeschwenkt und die Binden in eine Ablagemulde 122 mit halbrunder Kontur gelegt.

Anschließend wird die zu prüfende Binde 1 ein Stück abgewickelt und durch das Abzugswerk 110 und die Walzen des Zugwerks 310 durchgeführt. Zwischen Abzugswerk 110 und Zugwerk 310 hängt die Binde 1 durch. Anschließend wird der Anfang des bahnförmigen Materials in die Wickelwalze 320 eingelegt und eingeklemmt.

Die Messungen für die ungedehnte und gedehnte Länge laufen parallel ab. Das Ermitteln der rückgewonnenen Länge kann erst im Anschluss dazu erfolgen, da die Binde hier einer Belastungs- und Ruhephase über eine definierte Zeit ausgesetzt werden muss.

Die Höhe des Durchhanges der Binde zwischen Abzugswerk 110 und Zugwerk 310 wird mittels des optischen Durchhangssensors 220 ermittelt. Mittels dieser Messung wird die Winkelgeschwindigkeit der Abzugswalzen 111, 112 angepasst und konstant gehalten.

Zur Messung der ungedehnten Länge wird das Messradsystem 210 mit Messrad und Drehgeber eingesetzt. Das Messrad läuft direkt auf dem bahnförmigen Material 1 an einer unteren Walze 312a des Zugwerkes 310 entlang und wird durch Reibschluss angetrieben.

Die Zugkraft, die zur Bestimmung der gedehnten Länge aufgebracht werden muss, wird zwischen Zugwerk 310 und der Wickelwalze 320 aufgebaut. In Folge einer initialen Geschwindigkeitsdifferenz zwischen dem Zugwerk 310 und der Wickelwalze 320 wird die Binde 1 durch die daraus resultierende Kraftwirkung gedehnt. Ist die definierte Zugkraft erreicht, so wird die Zugkraft mittels eines Kraftmessers 350 konstant gehalten und die Binde 1 wird unter Wirkung dieser definierten Zugkraft gedehnt aufgewickelt. Bei Vliesbinden erfolgt die Dehnung mit einer Kraft von 3 N pro einem Zentimeter Bindenbreite. Binden aus Gewebe oder Gewirke werden mit einer Kraft von 10 N pro einem Zentimeter Bindenbreite gedehnt.

Die Länge der gedehnten Binde wird mittels des zweiten optischen Sensors 330 bestimmt.

Nachdem die gedehnte und die ungedehnte Länge der Binde bestimmt wurde, ist die Binde in gedehntem Zustand auf der Wickelwalze 320 aufgewickelt. Dort harrt die gedehnte Binde für eine Minute aus. Danach wird die Binde mittels des Zugwerks 310 abgezogen und verharrt dann in der Ruhemulde 340 für zwei Minuten im entspannten Zustand. Nach der Ruhephase wird die Binde 1 vom Zugwerk 310 in Richtung eines Abwickelgestells gefördert und mit dem Messradsystem 210 in dem zweiten Abschnitt II wird die rückgewonnene Länge des bahnförmigen Materials bestimmt.

Aus der Länge $l_g$ des gedehnten bahnförmigen Materials und der Länge $l_0$ des ungedehnten bahnförmigen Materials wird die Dehnung $\epsilon$ des bahnförmigen Materials folgendermaßen bestimmt:

$$\epsilon = \frac{l_g - l_0}{l_0} \cdot 100.$$

Die Dehnung wird in Prozent angegeben.

Der Rückzug R des bahnförmigen Materials wird ebenfalls in Prozent angegeben und wird berechnet mittels:

$$R = \frac{l_g - l_R}{l_g - l_0} \cdot 100$$

Dabei ist $l_R$ die rückgewonnene Länge des bahnförmigen Materials.

**Patentansprüche**

1. Prüfgerät zur Bestimmung der Dehnungseigenschaften von bahnförmigem, insbesondere textilem Material umfassend

   a. einen ersten Bereich (I), in dem das bahnförmige Material (1) in einem vorzugsweise gewickelten Lagerzustand bereitgestellt wird,
   b. optional einen zweiten Bereich (II), in dem ein Bestimmen der ungedehnten Länge des bahnförmigen Materials (1) erfolgen kann,
   c. einen dritten Bereich (III),

   welcher ein Zugwerk (310),
   eine Wickelwalze (320),
   und einen Längenmesssensor (330) umfasst,
   wobei das Zugwerk (310) dazu ausgestaltet ist, das bahnförmige Material zur Wickelwalze (320) weiterzuleiten,
   wobei das bahnförmige Material (1) unter Einwirkung einer vorherbestimmten Zugkraft zwischen Zugwerk (310) und Wickelwalze (320) gedehnt werden kann, wobei die Wickelwalze (320) dazu ausgestaltet ist, dass das bahnförmige Material (1) unter Wirkung der Zugkraft aufgewickelt werden kann,
   und die gedehnte Länge mittels des Längenmesssensors (330) bestimmt werden kann.

2. Prüfgerät nach Anspruch 1, wobei der Längenmesssensor (330) ein optischer Sensor ist.

3. Prüfgerät nach mindestens einem der vorigen Ansprüche, wobei der erste Bereich (I) ein ein Abzugswerk (110) umfasst, und wobei das Abzugswerk (110) dazu ausgestaltet ist, das bahnförmige Material (1) aus seinem gewickelten Lagerzustand in eine zumindest teilweise abgewickelte Materialbahn (1) zu überführen.

4. Prüfgerät nach mindestens einem der vorigen Ansprüche, wobei das Zugwerk (310) mindestens zwei Walzen (311, 312a, 312b) umfasst.

5. Prüfgerät nach Anspruch 4, wobei das Zugwerk (310) eine angetriebene Walze (312a, 312b) und eine Anpresswalze (311) umfasst,
   und wobei die Anpresswalze (311) dazu ausgestaltet ist, das bahnförmige Material (1) an die angetriebene Walze (312) anzupressen.

6. Prüfgerät nach mindestens einem der vorigen Ansprüche, wobei der zweite Bereich (II) ein Messradsystem (210) mit einem Drehgeber und einem Messrad umfasst und

wobei das Messradsystem (210) optional an dem bahnförmigen Material (1) an der angetriebenen Walze (312a) des Zugwerkes (310) läuft und mittels Reibschluss angetrieben wird.

7. Prüfgerät nach mindestens einem der vorigen Ansprüche, wobei der dritte Bereich (III) einen Kraftmesser (340) umfasst, welcher dazu ausgestaltet ist die Zugkraft zwischen Zugwerk (310) und Wickelwalze (320) zu bestimmen.

8. Prüfgerät nach mindesten einem der vorigen Ansprüche, wobei die Wickelwalze (320) ein mittels eines Motors angetriebener Zentrumswickler (320) ist und der Zentrumswickler (320) dazu ausgestaltet ist, das bahnförmige Material (1) in Form einer Archimedischen Spirale aufzuwickeln.

9. Verfahren zur Bestimmung der Dehnungseigenschaften eines bahnförmigen Materials (1) unter Verwendung des Prüfgeräts nach einem der Ansprüche 1 bis 8 umfassend die Schritte:

a. Einlegen eines zu prüfenden bahnförmigen Materials (1) in das Prüfgerät
b. Zumindest teilweises Abwickeln des zu prüfenden bahnförmigen Materials (1) und Einlegen eines Anfangsstücks des bahnförmigen Materials (1) in die Wickelwalze (320)
c. Zuführen des bahnförmigen Materials (1) an das Zugwerk (310) vom ersten Bereich (I)
d. Anbringen einer Zugkraft auf das bahnförmige Material (1) zwischen Zugwerk (310) und Wickelwalze (320) und gleichzeitiges Weiterführen des gedehnten bahnförmigen Materials (1) an die Wickelwalze (320)
e. Aufwickeln des gedehnten bahnförmigen Materials (1) unter Wirkung der Zugkraft auf der Wickelwalze (320)
f. Bestimmen der gedehnten Länge mittels des Längenmesssensors (330).

10. Verfahren nach Anspruch 9 unter Verwendung des Prüfgeräts nach Anspruch 6 umfassend die Schritte a bis f, wobei beim Zuführen des bahnförmigen Materials (1) an das Zugwerk (310) eine Messung der ungedehnten Länge mittels des Messrads mit Drehgeber durchgeführt wird.

11. Verfahren nach einem der Ansprüche 9 bis 10 unter Verwendung eines Prüfgeräts nach Anspruch 3, wobei das bahnförmige Material (1) dem Zugwerk (310) über einen Durchhang zwischen Abzugswerk (110) und Zugwerk (310) zugeführt wird.

12. Verfahren nach Anspruch 11, wobei der Durchhang mittels eines Durchhangssensors (220) bestimmt wird und der so bestimmte Durchhang zur Regelung der Winkelgeschwindigkeit der Walzen (111, 112) des Abzugswerks (110) verwendet wird.

13. Verfahren nach einem der Ansprüche 9 bis 12, wobei die Zugkraft mittels des Kraftmessers (340) kontrolliert wird.

14. Verfahren nach einem der Ansprüche 9 bis 13, wobei die Zugkraft konstant ist.

15. Verfahren nach einem der Ansprüche 9 bis 14, wobei die Zugkraft mittels Änderung der Walzengeschwindigkeiten des Zugwerks (310) und der Walzengeschwindigkeiten der Wickelwalze (320) angepasst werden kann.

16. Verfahren zum Ermitteln einer rückgewonnenen Länge des bahnförmigen Materials (1) unter Verwendung des Prüfgeräts nach Anspruch 6 bis 8, wobei

a. das bahnförmige Material (1) in gedehnten Zustand zunächst für eine vom Nutzer vorbestimmte Zeit auf der Wickelwalze (320) ausharrt,
b. das bahnförmige Material (1) in einer Ruhephase in entspanntem Zustand gelagert wird
c. das bahnförmige Material (1) mittels des Zugwerks (310) befördert wird und gleichzeitig die rückgewonnene Länge mittels des Messradssystems (210) bestimmt wird.

I                      III

330

310

1

320

100

**Abbildung 1**

Abbildung 2

Europäisches
Patentamt

European
Patent Office

Office européen
des brevets

# EUROPÄISCHER RECHERCHENBERICHT

Nummer der Anmeldung

EP 23 21 6329

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 116 609 185 A (SUQIAN BAIJING KNITTING CO LTD) 18. August 2023 (2023-08-18) * das ganze Dokument * ----- | 1-16 | INV. G01N3/08 |
| X | CN 114 739 798 A (ANHUI SIBODA NEW MAT CO LTD) 12. Juli 2022 (2022-07-12) * Zusammenfassung; Abbildung 2 * ----- | 1-16 | |
| X | DE 10 2007 033394 A1 (VOITH PATENT GMBH [DE]) 22. Januar 2009 (2009-01-22) * Zusammenfassung; Abbildungen 1, 2 * * Absätze [0026] - [0027], [0031], [0034] * ----- | 1-16 | |
| X | CN 116 465 735 A (SHOUGANG JINGTANG UNITED IRON & STEEL CO LTD) 21. Juli 2023 (2023-07-21) * Zusammenfassung; Abbildungen 2, 3 * ----- | 1-16 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 31. Mai 2024 | Giráldez Sánchez, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.......................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 23 21 6329

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

31-05-2024

| Im Recherchenbericht angeführtes Patentdokument | Datum der Veröffentlichung | Mitglied(er) der Patentfamilie | Datum der Veröffentlichung |
|---|---|---|---|
| CN 116609185 A | 18-08-2023 | KEINE | |
| CN 114739798 A | 12-07-2022 | KEINE | |
| DE 102007033394 A1 | 22-01-2009 | DE 102007033394 A1<br>WO 2009010343 A1 | 22-01-2009<br>22-01-2009 |
| CN 116465735 A | 21-07-2023 | KEINE | |

EPO FORM P0461

Für nähere Einzelheiten zu diesem Anhang : siehe Amtsblatt des Europäischen Patentamts, Nr.12/82